# EUROPEAN PATENT SPECIFICATION

(11) **EP 3 029 588 B1**
(45) Date of publication and mention of the grant of the patent: **18.09.2019**
(21) Application number: 15197696.6
(22) Date of filing: 03.12.2015
(51) Int. Cl.: G16H 40/63, G16H 50/20, G01N 33/86

(54) **BLOOD TESTING SYSTEM RESULT INTERPRETER INTERFACE AND METHODS**
SCHNITTSTELLE FÜR INTERPRETIERER DER ERGEBNISSE EINES BLUTUNTERSUCHUNGSSYSTEMS UND VERFAHREN
PROCÉDÉS ET INTERFACE INTERPRÈTE DE RÉSULTAT DE SYSTÈME D'ANALYSE DE SANG

(30) Priority: 05.12.2014 US 201414562332
(43) Date of publication of application: 08.06.2016
(73) Proprietor: C A Casyso GmbH, 4052 Basel (CH)
(72) Inventor: GÖRLINGER, Klaus, 85551 Kirchheim (DE)
(74) Representative: Isarpatent

(56) References cited:
- EP-A1- 2 538 360
- US-A1- 2002 099 283
- US-A1- 2011 252 352
- James S Bennett ET AL: "Experience Using EMYCIN", Infotech Ltd, 1 January 1981 (1981-01-01), page 314, XP055271814, Retrieved from the Internet: URL:http://aitopics.org/sites/default/file s/classic/Buchanan/Buchanan18.pdf [retrieved on 2016-05-11]
- MICHAEL T. GANTER ET AL: "Coagulation Monitoring: Current Techniques and Clinical Use of Viscoelastic Point-of-Care Coagulation Devices", ANESTHESIA & ANALGESIA, vol. 106, no. 5, 1 May 2008 (2008-05-01), pages 1366-1375, XP055039818, ISSN: 0003-2999, DOI: 10.1213/ane.0b013e318168b367
- A. H. STAMMERS ET AL: "Point-of-care coagulation monitoring: applications of the thromboelastograph", ANAESTHESIA, vol. 53, no. S2, 1 May 1998 (1998-05-01), pages 58-59, XP055039832, ISSN: 0003-2409, DOI: 10.1111/j.1365-2044.1998.tb15159.x

## Description

### TECHNICAL FIELD

This document relates to systems and methods for testing characteristics of a blood sample, such as an automated thromboelastometry system for blood coagulation analysis and other analytical systems for testing blood.

### BACKGROUND

Hemostasis is the human body's response to blood vessel injury and bleeding. Hemostasis involves a coordinated effort between platelets and numerous blood clotting proteins (or clotting factors), resulting in the formation of a blood clot and the subsequent stoppage of bleeding.

One group of tests to assess the potential of blood to form an adequate clot are known as "viscoelastic methods." In at least some viscoelastic methods, the blood clot firmness (or other parameters dependent thereon) is determined over a period of time, for example, from the formation of the first fibrin fibers until the dissolution of the blood clot by fibrinolysis. Blood clot firmness is a functional parameter which contributes to hemostasis *in vivo,* as a clot must resist blood pressure and shear stress at the site of vascular injury or incision. In many cases, clot firmness may result from multiple interlinked processes including coagulation activation, thrombin formation, fibrin formation and polymerization, platelet activation, and fibrin-platelet interaction.

Viscoelastic method test result data and the result data from other types of analytical blood tests are sometimes used by medical practitioners for determining patient diagnoses, or for identifying potential health conditions for further investigation. In some scenarios, the medical practitioner may be required to assess two or more types of blood test result data (e.g., data from two or more different blood testing machines), which can be complex and time-consuming in medical emergency situations. Moreover, different hospitals or other medical care facilities may impose different standards or responses to the same blood test result data, thereby further increasing the complexity imposed upon the medical practitioner attempting to assess the blood test result data from two or more different blood testing machines.

EP 2 538 360 A1 describes a method of predicting a blood dilution risk value using a mathematical model, as a result of which a time development of said risk value is displayed to a user, wherein said risk value describes a risk of clotting and/or embolism and/or bleeding.

Scientific publication "Experience using EMYCIN", James S. Benet et al., Infotech Ltd., 1 January 1981, page 314, XP055271814, retrieved from the Internet: "http://aitopics.org./sites/default/files/classic/buchanan/buchananl8.pdf", retrieved on 2016-05-11, paragraph 16.2 "CLOT: A consultant for bleeding disorders" and 16.3 "The CLOT knowledge base", describes experiences by a group of scientists working on a prototype of a virtual consultant for identifying the presence and type of a bleeding defect in a patient.

### SUMMARY

The preferred embodiment described herein provides a system configured to receive blood test result data (from one or more blood analyzer machines) and, based on the blood test result data, to provide a result interpretation output via a user interface to assist the user in identifying a relevant diagnosis or potential health conditions for further investigation. As used herein, the term "result interpretation output" means a communication output via a user interface that, based upon the blood test result data received from one or more blood analyzer machines that analyzed a blood sample, identifies a suggested diagnosis or potential health condition for a patient from which the blood sample was obtained. The "result interpretation output" is different from the actual blood test result data received from a blood analyzer machine, and instead provides a computer-driven interpretation of the blood test result data. Accordingly, in a preferred implementation, a blood analyzer machine is configured not only to perform test operations on a blood sample and provide blood test result data, but also to provide a result interpreter interface that compares the various data points from the blood test result data to previously stored threshold values and thereby outputs to the user one or more meaningful messages to the user that identify a relevant diagnosis of the patient or potential health conditions for further investigation. Optionally, in particular embodiments, the previously stored threshold values are user-customizable in that a machine operator can modify the default threshold values in accordance with the standards of a particular hospital or other medical care facility.

Particular embodiments of a system described herein include a computer-implemented algorithm (optionally, implemented by a control console of a blood analyzer machine) that is configured to generate a result interpretation output based at least in part on the blood test result data received from one or more blood analyzer machines that analyzed a particular blood sample. Such a system may optionally include any of the features described in the text below.

An embodiment described herein includes a method that may include one or more of the following steps. The method includes the step of receiving, at a blood analyzer machine, user input indicative of a clinical setting of a patient that provided a blood sample. The method also includes the step of receiving, at the blood analyzer machine, the blood sample of the patient. The method also includes the step of performing, by the blood analyzer machine, one or more blood characteristic tests on the blood sample for providing blood test result data. The method includes the step of interpreting, by the blood analyzer machine and using an algorithm from a plurality of algorithms accessible to the blood analyzer machine, the blood test result data provided from the one or more blood characteristic testsThe method includes the step of displaying, using a user interface of the blood analyzer machine, one or more messages indicative of a potential diagnosis or health condition based on said interpreting the blood test result data provided from the one or more blood characteristic tests. The blood analyzer machine selects a particular algorithm of a plurality of the algorithms for interpreting the blood test result data in response to the user input indicative of the clinical setting of the patient. Said interpreting by the blood analyzer machine comprises analyzing the blood test result data in comparison to one or more preset algorithm-specific threshold values provided in a risk matrix using the algorithm so as to determine said one or more messages. The method also includes the step of receiving at the blood analyzer machine, via one or more data cables or via a wireless network connection for transferring data to the blood analyzer machine, external test result data from one or more external blood analyzer devices that are different from and remote from said blood analyzer machine, said one or more external blood analyzer devices being connected to the blood analyzer machine, the external test result data from the one or more external blood analyzer devices resulting from additional blood characteristic tests on the blood sample by the one or more external blood analyzer devices. Said interpreting by the blood analyzer machine includes analyzing, using said algorithm, the blood test result data from the blood analyzer machine and the external test result data from the one or more external blood analyzer devices. The one or more messages displayed by the blood analyzer machine are based on said interpreting the blood test result data from the blood analyzer machine and the external test result data from the one or more external blood analyzer devices. The interpreting further comprises comparing and contrasting the test results with each other using a computerized algorithm.

Such a method may optionally include one or more of the following features. In some embodiments, the one or more messages determined and displayed by the blood analyzer machine are a result interpretation output that is different from blood test result data output from the blood analyzer machine. The blood analyzer machine may comprise a thromboelastometry system including a movable probe to interact with at least a portion of the blood sample for measuring blood coagulation characteristics. In the embodiments, the method further includes the step of receiving, at the blood analyzer machine, external test result data from one or more external blood analyzer devices that are different from and remote from said blood analyzer machine. The external test result data from the one or more external blood analyzer devices may result from additional blood characteristic tests on the blood sample by the one or more external blood analyzer devices.

In the embodiments, the interpreting by the blood analyzer machine includes analyzing, using the algorithm, the blood test result data from the blood analyzer machine and the external test result data from the one or more external blood analyzer devices. The one or more messages displayed by the blood analyzer machine may be based on the interpreting of the blood test result data from the blood analyzer machine and the external test result data from the one or more external blood analyzer devices. In some embodiments, the one or more external blood analyzer devices comprises a blood gas analyzer configured to perform a blood characteristic test on the blood sample. In particular embodiments, the one or more external blood analyzer devices are connected to the blood analyzer machine via one or more data cables or a wireless network connection for transferring data to the blood analyzer machine. The method may optionally include, after displaying the one or more messages, receiving, at the blood analyzer machine, a user selection of a selectable element for changing a display mode of the user interface. Some such methods may include, in response to receiving the user selection, changing from displaying the one or more messages to displaying graphical or numeric information corresponding to at least some of the blood test result data.

In the embodiments of the method, the interpreting by the blood analyzer machine comprises analyzing the blood test result data in comparison to one or more preset threshold values provided in a risk matrix using the algorithm so as to determine said one or more messages. In some embodiments of the method, the one or more present threshold values are customizable by an operator of the machine so as to modify one or more default threshold values to one or more customized threshold values. Additionally, or alternatively, in some embodiments the blood analyzer machine comprises a control console including a user interface display device, one or more computer processors, and a computer-readable memory device. The plurality of algorithms accessible to the blood analyzer machine and the one or more present threshold values may be stored by the computer-readable memory device in some embodiments.

Various embodiments described herein include a blood analyzer system. The blood analyzer system may include a thromboelastometry testing device including a movable probe to interact with at least a portion of a blood sample for measuring blood coagulation characteristics of the blood sample. In some embodiments, the blood analyzer system also includes a user interface comprising a display device configured to display blood test result data indicative of the blood coagulation characteristics of the blood sample. The blood analyzer system may also include one or more processors and a computer memory device storing computer-readable instructions for one or more algorithms for interpreting the blood test result data. In some embodiments, the computer-readable instructions, when executed by the one or more processors, are configured to cause the system to perform at least some of the following operations: analyzing the blood test result data indicative of the blood coagulation characteristics; and outputting via the user interface one or more messages indicative of a potential diagnosis or health condition based on the analysis of the blood test result data.

Some embodiments of the blood analyzer system may optionally include one or more of the following features. The computer-readable instructions, when executed by the one or more processors, may be configured to cause the system to prompt a user for input indicative of a clinical setting of patient from which the blood sample is drawn. The computer-readable instructions, when executed by the one or more processors, are configured to cause the system to select a particular algorithm of a plurality of the algorithms for interpreting the blood test result data in response to the user input indicative of the clinical setting of the patient. In particular embodiments, the computer-readable instructions, when executed by the one or more processors, may be configured to cause the system to receive external test result data from one or more external blood analyzer devices that are different from and remote from said blood analyzer system. The blood analyzer system may comprise a data connection for receiving the external test result data from the one or more external blood analyzer devices. In particular embodiments, the data connection comprises a data cable connection or a wireless network connection. In some embodiments, the one or more messages indicative of a potential diagnosis or health condition are a result interpretation output that is displayed by the display device and that is different from blood test result data.

Other embodiments described herein include a blood analyzer system. In some embodiments, the blood analyzer system includes a blood test device that performs one or more blood characteristic tests on a blood sample for generating blood test result data, and a user interface that provides a result interpretation output based upon the blood test result data from the one or more blood characteristic tests. Some embodiments of the blood analyzer system may optionally comprise a thromboelastometry testing device including a movable probe to interact with at least a portion of the blood sample. The user interface of the blood analyzer system may include a display device of a control console that houses at least a portion of the thromboelastometry testing device. In particular embodiments, the result interpretation output may comprise one or more messages indicative of a potential diagnosis or health condition displayed by a display device of the user interface. The one or more messages may be different from blood test result data.

Particular embodiments described herein include a method for providing a result interpretation output using blood test result data of blood analyzer machine. The method may include one or more of the following steps. Some embodiments include the step of receiving blood test result data generated from one or more blood characteristic tests performed on a blood sample by a blood test device. Particular embodiments include the step of outputting, via a user interface, a result interpretation output based upon the blood test result data from the one or more blood characteristic tests. The result interpretation output is different from the blood test result data, in some embodiments. In some embodiments, the blood test device may comprise a thromboelastometry testing device including a movable probe to interact with at least a portion of a blood sample for measuring blood coagulation characteristics of the blood sample. In particular embodiments, the user interface may comprise a display device of a control console that houses at least a portion of the thromboelastometry testing device.

Some or all of the embodiments described herein may provide one or more of the following advantages. First, some embodiments of the blood analyzer system provided herein can be used in the clinical setting to reduce clinical decision-making errors, for example, by a clinician attempting to assess blood test result data (possibly from two or more different blood testing machines) in a medical emergency situation. The performance of clinical tests on a patient's blood sample, for example, can result in an overwhelming amount of information (e.g., data and test results from multiple tests). In such a case, a clinician may find it challenging to contemporaneously evaluate and interpret the extensive information so as to arrive at a proper diagnosis or conclusion. That can be especially true in the context of less experienced clinicians. Particular embodiments of the system described herein can assist the clinician by running computerized algorithms that may result in the determination of messages that may include recommendations for patient treatment or further testing. Such messages are displayed to the user on a user interface of the system. However, decision-making is done by the attending physician and recommendations are provided merely as information to assist the attending physician with the decision-making.

Second, some embodiments of the blood analyzer system described herein can advantageously facilitate standardization within a medical care facility (e.g., a hospital, a clinic, or the like) that controls the blood analyzer system. That is, each owner/operator or the blood analyzer system can create a customized protocol for analysis of blood test result data, customized test parameter threshold values, and customized messages that may include recommendations for patient treatment or further testing. Thus, although the blood analyzer system may be equipped with default protocols, threshold values, and recommendation outputs, each hospital can modify the default values so as to create a hospital-specific (e.g., specific to the hospital or other medical care facility) standard, thereby removing user-to-user variability and enhancing systemic process controls within the hospital or other medical care facility.

Third, use of the blood analyzer system described herein can, in particular embodiments, advantageously save time in a clinical setting, especially during a medical emergency situation in which a patient is bleeding. The time savings can be realized, for example, by automation of the analysis of multiple test results to arrive at a computer-generated message that may include a recommendation or suggested diagnosis to be considered by a clinician (as compared to a manual analysis). Such a time savings can be especially important in an urgent clinical setting (e.g., when timely action is needed because a patient's health or life is at risk). In some cases, a time savings from the automation of the analysis of multiple test results can also result in a reduction of patient care costs.

Fourth, use of the blood analyzer system described herein can, in various embodiments, advantageously improve the quality of care. Such quality of care improvements can result from, for example, implementation of clinical best practices into computer-automated algorithms that are embodied integrally with the blood analyzer system when implementing the result interpretation interface, as well as from standardization and error reduction as described above.

Further advantages associated with the blood analyzer systems described herein are also envisioned, as will be evident from the following disclosure.

The details of one or more embodiments of the invention are set forth in the accompanying drawings and the description below. Other features, objects, and advantages of the invention will be apparent from the description and drawings, and from the claims.

### DESCRIPTION OF DRAWINGS

FIG. 1 is a schematic system diagram showing a first blood analyzer machine that is networked with other blood analyzer machines in accordance with some embodiments.
FIG. 2 is a flowchart of a process for operating a system (such as the system of FIG. 1) having a result interpretation interface for interpreting blood test result data.
FIG. 3 is an example user interface display that can be used in conjunction with the system shown in FIG. 1.
FIG. 4 is an example user interface display that identifies a suggested diagnosis or potential health condition for a patient.
FIG. 5 is a flowchart of a computer-implemented cardiovascular algorithm that can be used in conjunction with the system of FIG. 1 and the process of FIG. 2.
FIG. 6 is another example user interface display that identifies a suggested diagnosis or potential health condition for a patient.
FIG. 7 is a flowchart of a computer-implemented trauma algorithm that can be used in conjunction with the system of FIG. 1 and the process of FIG. 2.
FIG. 8 is another example user interface display that identifies a suggested diagnosis or potential health condition for a patient.
FIG. 9 is a flowchart of a computer-implemented liver/GI algorithm that can be used in conjunction with the system of FIG. 1 and the process of FIG. 2.
FIG. 10 is a flowchart of a computer-implemented severe bleeding algorithm that can be used in conjunction with the system of FIG. 1 and the process of FIG. 2.
FIG. 11 is a flowchart of a computer-implemented postpartum hemorrhage algorithm that can be used in conjunction with the system of FIG. 1 and the process of FIG. 2.
FIG. 12 is an example of a graphic that quantifies the firmness of a blood clot during clot formation, as calculated and displayed by a blood analyzer machine.
FIG. 13 is a perspective view of an example blood analyzer machine that is displaying a collection of graphics that quantify the firmness of a blood clot during clot formation.
FIG. 14 is an example risk matrix that includes test parameter threshold values and recommended tests for use in conjunction with the algorithms described herein.

Like reference symbols in the various drawings indicate like elements.

### DETAILED DESCRIPTION OF ILLUSTRATIVE EMBODIMENTS

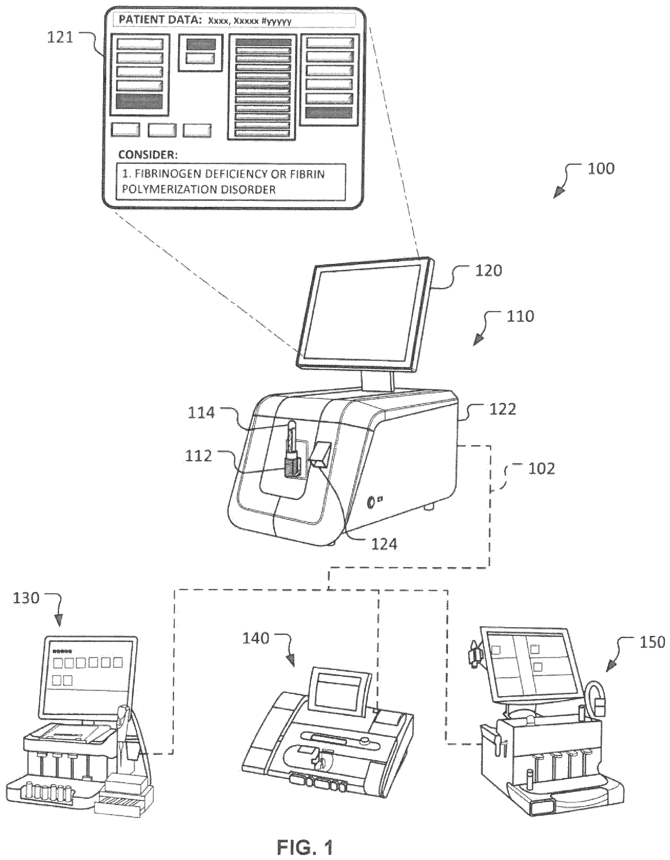

Referring to **FIG. 1**, embodiments of a blood testing system 100 include a first blood analyzer machine 110 (e.g., a thromboelastometry analyzer console in this depicted embodiment) that includes a results interpretation interface configured to receive blood test result data (generated from the blood test performed by the console) and, based on the blood test result data, to identify a relevant diagnosis or potential health conditions for further investigation. Optionally, the thromboelastometry analyzer console 110 may be connected, via a data connection 102 (e.g., a data communication network in this embodiment, or a direct data cable connection or a wireless communication network in other embodiments), with one or more other blood analyzer devices. In the depicted blood testing system 100, the thromboelastometry analyzer console 110 is connected with a second thromboelastometry analyzer console 130, a blood gas analyzer 140, and a platelet aggregation analyzer 150, but other blood analyzer devices are also contemplated herein.

While in the depicted example blood testing system 100 the thromboelastometry analyzer console 110 is connected in the data communication network 102 with three additional analytical testing devices, in some implementations zero, one, two, four, five, six, seven, or more than seven other analytical testing devices are networked with the thromboelastometry analyzer console 110. Any and all such implementations are within the scope of this disclosure. It should also be understood that, while the example blood testing system 100 is depicted as including particular types of analytical testing devices, the blood testing system 100 is provided as an illustrative example that is representative of all potential combinations of different analytical testing devices of any and all kinds. For example, in some embodiments additional types of analytical testing devices such as, but not limited to, a system for measuring activated coagulation time (ACT) and/or hemodynamic monitors can be included as part of the blood testing system 100.

In the depicted embodiment, the thromboelastometry analyzer console 110, the second thromboelastometry analyzer console 130, the blood gas analyzer 140, and the platelet aggregation analyzer 150 can communicate with each other via the data communication network 102. For example, in some embodiments test results from the second thromboelastometry analyzer console 130, the blood gas analyzer 140, and/or the platelet aggregation analyzer 150 can be communicated to the first thromboelastometry analyzer console 110 via the data communication network 102.

The data communication network 102 can be implemented using any type of networking technology that is capable of facilitating communications between the analytical testing devices of blood testing system 100. In some embodiments the data communication network 102 is a computer data network such as, but not limited to an intranet, the internet, a LAN, and the like, and combinations thereof. In some embodiments, portions or all of the data communication network 102 is a hard-wired network. In some embodiments, portions or all of the data communication network 102 is a wireless network (e.g., Wi-Fi, Bluetooth, NFC, RF, IR, and the like). In particular embodiments, the data communication network 102 comprises a combination of hard-wired and wireless network connections.

In some embodiments, the thromboelastometry analyzer console 110 performs a coordination and overall analysis role within the blood testing system 100. For example, in some embodiments one or more analytical testing devices within the blood testing system 100 communicates one or more test results to the thromboelastometry analyzer console 110. Thereafter, the thromboelastometry analyzer console 110 performs a results interpretation analysis using the test results received from the other analytical testing devices within the blood testing system 100, in conjunction with the test results generated from the thromboelastometry analyzer console 110 itself. In that fashion, the blood testing system 100 facilitates performance of a range of analytical tests, in some cases by multiple testing devices, and the test results can potentially be compared and contrasted to each other using a computerized algorithm to thereby enhance the analytical capabilities of the blood testing system 100. In such a case, blood samples from the same patient are run in the various analytical testing devices within the blood testing system 100. The test results from the patient's sample(s) are then communicated to the thromboelastometry analyzer console 110 for performance of the computerized algorithm.

In some scenarios, as described further below, the performance of the computerized algorithm may yield a recommended action for treating the patient, and/or for further testing, and the like. Such a recommendation(s) may be displayed on the user interface display 120 of the thromboelastometry analyzer console 110. However, decision-making is done by the attending physician and such recommendations are provided merely to assist the attending physician. In some embodiments, the user interface display 120 is a touchscreen display that can receive user inputs (as described further below).

In some implementations, the blood testing system 100 includes just the first blood analyzer machine 110 itself. Or, while the blood testing system 100 may include other test devices in addition to the first blood analyzer machine 110, in some situations no relevant test results from the other test devices are available to the console 110. Nevertheless, as described further below, because the first blood analyzer machine 110 can perform multiple tests, the blood test result data generated from the multiple tests performed by the first blood analyzer machine 110 can be compared/contrasted with each other in a results interpretation analysis performed by first blood analyzer machine 110. Hence, it should be understood that while in some embodiments the results interpretation analysis performed by first blood analyzer machine 110 is performed using test results from other test devices in addition to results from the analyzer console 110, in some embodiments the results interpretation analysis is performed using test results from the analyzer console 110 exclusive of test results from other test devices.

In some implementations, the first blood analyzer machine 110 (of any type) performs an results interpretation analysis using the blood test result data from one or more of the analytical testing devices within the blood testing system 100. Alternatively, in some implementations an additional computer that is external to the first blood analyzer machine 110 performs the results interpretation analysis. That is, in some implementations an additional computer (not shown) is included within the blood testing system 100, and the additional computer performs the results interpretation analysis described herein.

Still referring to FIG. 1, in this embodiment, the thromboelastometry analyzer console 110 is configured to determine a number of blood coagulation characteristics of a blood sample that is input into a cartridge 112. However, such a cartridge 112 is not required in some embodiments of thromboelastometry analyzers that can be used to perform the results interpretation analysis described herein. In some embodiments, the cartridge 112 is configured as a single-use cartridge that includes a blood sample receiver for mating with a blood sample reservoir 114 (e.g., a vacutainer sample tube supplied by Becton, Dickinson & Company of Franklin Lakes, NJ, or another blood reservoir structure). In some cases, an adapter may be used to couple other types of blood sample reservoirs 114 with the cartridge 112 (e.g., tubing may be used through which blood can be injected into the cartridge 112, and the like). The thromboelastometry analyzer console 110 can be used as a whole blood coagulation analysis system that is particularly advantageous at a point-of-care site (e.g., in a surgical theater while a patient is undergoing or preparing for surgery, or the like). Additionally, thromboelastometry analyzer console 110 can be used as a whole blood coagulation analysis system in a laboratory setting.

In some embodiments, the thromboelastometry analyzer console 110 includes a probe or pin that can be removably positioned within a cup of the cartridge 112 (or another type of blood sample container). The cup may contain a blood/reagent mixture. A clearance space exists between the probe and cup. In some embodiments, the probe is oscillated rotationally, back and forth, by about +/- 5°. The oscillations are measured, and as the blood/reagent begins to become firmer because of thrombolysis, the oscillations are reduced. The measurements, by the thromboelastometry analyzer console 110, of such oscillations over a period of time thereby generates thromboelastometry results.

The example thromboelastometry analyzer console 110 includes the user interface 120 (with touchscreen display in this embodiment) and a main chassis 122. The user interface display 120 can be configured to output one or more graphical results from the blood testing assays performed via the cartridge 112 (e.g., one or more plots, such as those sometimes refer to as a TEMogram, numeric data or measurements, or a combination thereof). In some embodiments, the user interface display 120 is rigidly attached to the analyzer console 122. In particular embodiments, the user interface display 120 is pivotable and/or is otherwise positionally adjustable in relation to the main chassis 122.

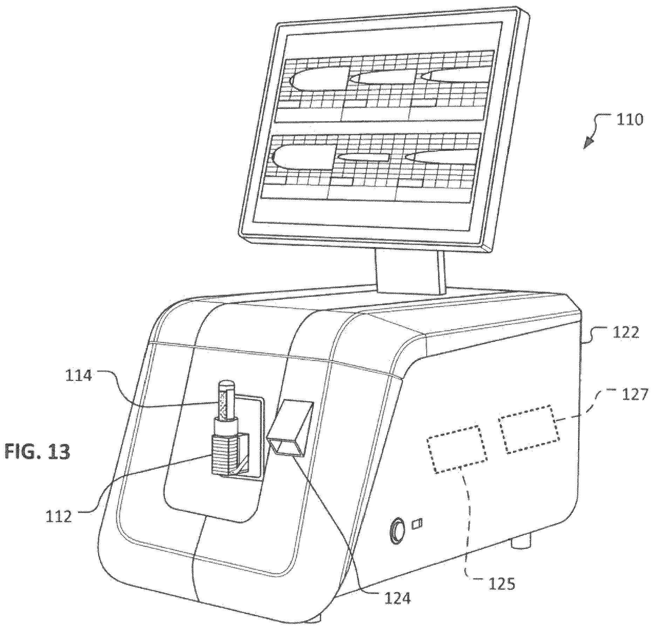

The main chassis 122 of the thromboelastometry analyzer console 110 houses the hardware devices and sub-systems that control the operations of the analyzer console 110 and that facilitate communications with other blood analyzer devices over a network (e.g., data communication network 102). For example, the analyzer console 110 houses one or more processors 125 (refer to FIG. 13). In addition, the analyzer console 110 houses memory devices that can store an operating system and other executable instructions. For example, the analyzer console houses memory devices 127 that can store computer-readable instructions for one or more algorithms for interpreting the blood test result data of the blood testing system 100.

In some embodiments, the computer-readable instructions, when executed by the one or more processors 125, are configured to cause the system to perform operations such as analyzing of the blood test result data indicative of the blood coagulation characteristics, and outputting via the user interface 120 one or more messages indicative of a potential diagnosis or health condition based on the analysis of the blood test result data. Further, in some embodiments the computer-readable instructions stored in the memory devices 127, when executed by the one or more processors 125, are configured to cause the analyzer console 110 to prompt a user for input indicative of a clinical setting of patient from which the blood sample is drawn.

As described further below, in some embodiments the computer-readable instructions stored in the memory devices 127, when executed by the one or more processors 125, are configured to cause the thromboelastometry analyzer console 110 to select a particular algorithm of a plurality of the algorithms for interpreting the blood test result data in response to the user input indicative of the clinical setting of the patient. In addition, in some embodiments the computer-readable instructions stored in the memory devices 127, when executed by the one or more processors 125, are configured to cause the thromboelastometry analyzer console 110 to receive external test result data from one or more external blood analyzer devices (e.g., the second thromboelastometry analyzer console 130, the blood gas analyzer 140, and the platelet aggregation analyzer 150) that are different from and remote from thromboelastometry analyzer console 110.

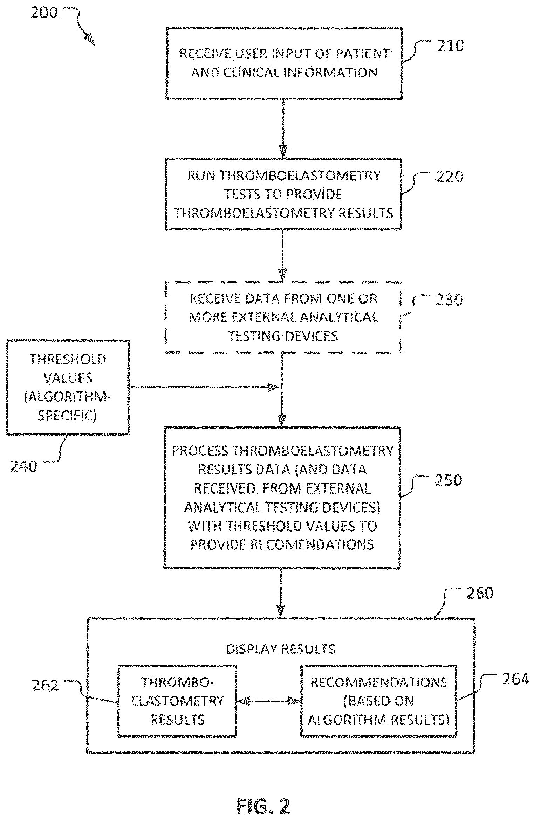

Referring now to **FIG. 2**, in some implementations the blood testing system 100 can be operated in accordance with an example process 200. In particular, the first blood analyzer machine 110 (e.g., a thromboelastometry analyzer console in this embodiment, but a different type of blood analyzer machine in other embodiments), in its coordination and overall analysis role within the blood testing system 100, can perform the process 200. For example, as previously described, the thromboelastometry analyzer console 110 can implement the process 200 using the one or more processors 125 to execute computer-readable instructions stored on the memory device 127 (housed in the main chassis 122; refer to FIG. 13) for the algorithms for interpreting the blood test result data. As such, the blood testing system 100 can be configured to receive blood test result data (generating using one or more blood testing devices) and, based on the blood test result data, to provide a result interpretation output via a user interface to assist the user in identifying a relevant diagnosis or potential health conditions for further investigation.

At step 210, patient identification and clinical information can be entered into the thromboelastometry analyzer console 110. In the depicted embodiment, such information can be entered by a clinician using, for example, the user interface display 120. That is, the touchscreen display 120 is configured to receive user input and to display output information to the user. For example, the user can enter information to the thromboelastometry analyzer console 110 by making selections of various soft-buttons that may be displayed on the touchscreen display 120 at times during the beginning, middle, and end of the process 200. In some embodiments, other selections such as, but not limited to, soft keyboard entries can be provided via touchscreen display 120.

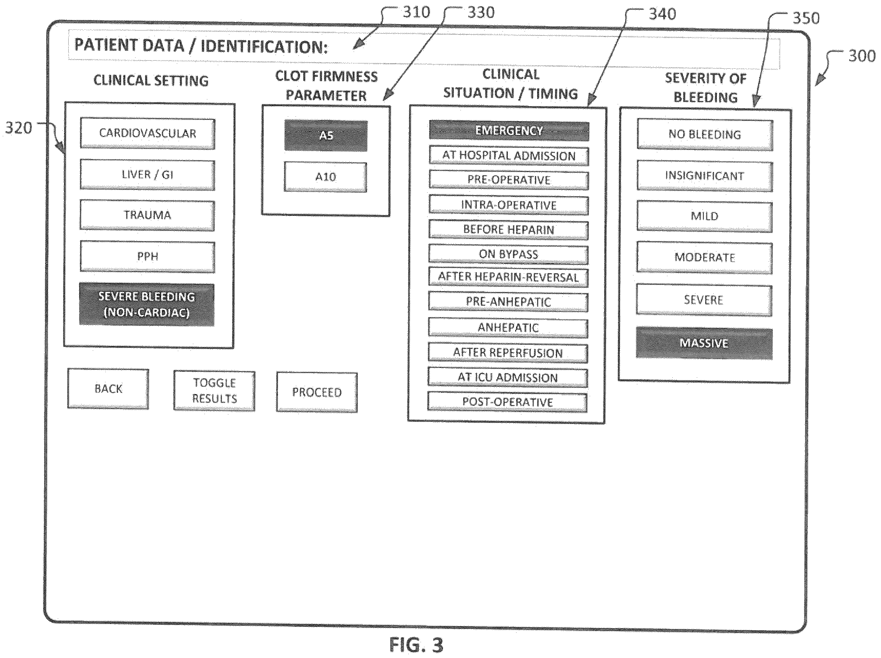

One example implementation of step 210 is illustrated in a screen shot 300 shown in FIG. 3. The screen shot 300 can be implemented, for example, on the touchscreen display 120, or on another user interface that is part of the blood testing system 100.

The patient information can be entered in a field 310 entitled "Patient Data / Identification:". The patient information entered can include, for example, patient name, patient birth date, patient ID number (e.g., numeric or alpha-numeric), blood sample ID number, and the like. Data may be entered using a keyboard, soft keyboard, barcode scanner, and the like.

In some embodiments, the clinical information can be entered, for example, by making selections from a clinical setting selection menu 320, a clot firmness parameter selection menu 330, a clinical situation / timing selection menu 340, and a severity of bleeding selection menu 350. In the depicted implementation, the clinical information is entered via touchscreen selections.

The clinical information entered during step 210 can include, for example, the clinical setting (e.g., cardiovascular surgery, trauma, liver surgery or GI bleeding, postpartum hemorrhage (PPH), and the like) as displayed in the clinical setting selection menu 320. In the depicted example, "Severe Bleeding (non-cardiac)" has been selected (as identified by the reverse highlighting). A clot firmness parameter can be selected from the clot firmness parameter selection menu 330. In the depicted example, "A5" has been selected (as identified by the reverse highlighting). Additionally, in some embodiments the clinical information entered during step 210 can include the clinical situation (e.g., emergency, at hospital admission, pre-operative, intra-operative, before heparin, on bypass, after heparin-reversal, pre-anhepatic, anhepatic, after reperfusion, at ICU admission, post-operative, and the like) as displayed in the clinical situation / timing selection menu 340. In the depicted example, "Emergency" has been selected (as identified by the reverse highlighting). Still further, in some embodiments the clinical information entered during step 210 can include the severity of bleeding (e.g., no bleeding, insignificant, mild, moderate, severe, massive, and the like) as displayed in the severity of bleeding selection menu 350. In the depicted example, "Massive" has been selected (as identified by the reverse highlighting). In some embodiments, other types of clinical information may additionally, or alternatively, be entered during step 210.

In some embodiments, the data entry at step 210 can be performed additionally or alternatively by user voice entry. In other embodiments, the user interface of thromboelastometry analyzer console 110 may include other peripheral devices (e.g., a mouse, a keyboard, an additional display device, and the like) that can be used to input patient and clinical information. In some embodiments, the data communication network 102 may be used to allow for remote devices to input information to the thromboelastometry analyzer console 110. For example, in some embodiments one or more remote displays can be utilized via network connections. In some embodiments, the thromboelastometry analyzer console 110 also includes an external barcode reader 124. The external barcode reader 124 can facilitate convenient one-dimensional or two-dimensional barcode entry of data such as, but not limited to, patient identification data, clinical information, blood sample data, user identification, normal values, and the like. Alternatively or additionally, the thromboelastometry analyzer console 110 can be equipped with a reader configured to read near-field communication tags, RFID tags, or the like, to provide for convenient input of data into analyzer console 110.

Still referring to FIG. 2, at step 220 the thromboelastometry tests are run by the thromboelastometry analyzer console 110, and corresponding results from the thromboelastometry tests are determined. In some implementations, the thromboelastometry tests are run by the performance of two or more test process cycles of the same thromboelastometry analyzer console 110. In some implementations, the thromboelastometry tests are run by the performance of a single test process cycle of the thromboelastometry analyzer console 110. Additionally or alternatively, blood test result data from one or more test cycles performed by one or more other blood analyzer machines (which are performed contemporaneously with, or sequentially before or after, the test cycles performed by the thromboelastometry analyzer console 110) can be communicated to the thromboelastometry analyzer console 110.

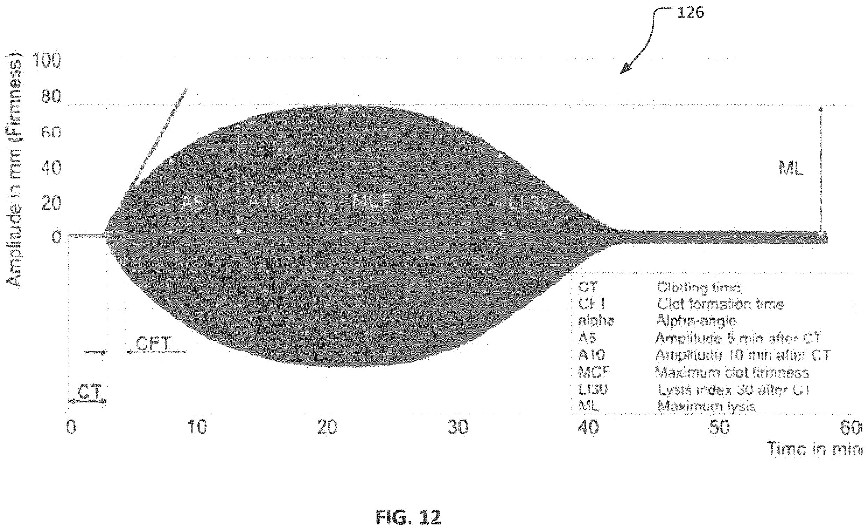

Thromboelastometry and thromboelastography are based on the measurement of the elasticity of blood by continuous graphic logging of the firmness of a blood clot during clot formation (e.g., pertaining to coagulation factors and inhibitors, platelets and fibrin) and subsequent fibrinolysis. An example of such a continuous graphic logging 126 of the firmness of a blood clot during clot formation, as calculated and optionally displayed by the thromboelastometry analyzer console 110, is shown in FIG. 12. FIG. 13 illustrates the thromboelastometry analyzer console 110 displaying multiple such graphic loggings of the firmness of a blood clot during clot formation.

In some embodiments, the thromboelastometry analyzer console 110 facilitates determination of various thromboelastometry parameters such as, but not limited to, clotting time, clot formation time, alpha angle, amplitude, maximum clot firmness, lysis onset time, lysis time, lysis index (%), and maximum lysis (%). Those example parameters are described in **TABLE 1** below:

**TABLE 1**

| **Parameter** | **Definition** | **Interpretation** |
|---|---|---|
| Clotting Time (CT) [sec] | The time from test start until first significant level of clot firmness (2 mm) is reached. | Normal/reduced/increased speed of coagulation initiation. |
| Clot Formation Time (CFT) [sec] | The time from the CT until a clot firmness of 20 mm is reached. | Normal/reduced/increased speed of coagulation amplification and propagation |
| Alpha Angle (α) | Angle between the baseline and a tangent to the clotting curve through the 2 mm (CT) point. | Normal/reduced/increased speed of coagulation amplification and propagation |
| A5, A10, A20 Amplitude [mm] | Clot firmness (amplitude) at the time points 5, 10, 20 minutes after CT. | Normal/reduced/increased clot firmness. |
| Maximum Clot Firmness (MCF) [mm] | Maximum clot firmness (maximum amplitude) of the developed clot during the test. | Normal/reduced/increased clot firmness. |
| Lysis Onset Time (LOT) [sec] | The time after CT when significant lysis starts. | Onset of clot lysis |
| Lysis Time (LT) [sec] | The time after CT when lysis is complete | Completion of clot lysis |
| LI30, LI45, LI60 Lysis Index [%] | Percentage of lysis in proportion to MCF at the time points 30, 45, 60 minutes after CT. | Degree of clot lysis |
| Maximum Lysis (ML) [%] | Lowest clot firmness in percent of the MCF in course of the test run | Degree of clot lysis |

In addition to the parameters above, the thromboelastometry analyzer console 110 may perform certain assays to monitor and analyze different aspects of the coagulation state of a blood sample in order to assist in the assessment of patient clinical hemostasis conditions. For example, in some embodiments the ROTEM® thromboelastometry system can perform at least four of five different assays: INTEM, HEPTEM, EXTEM, FIBTEM, and APTEM. Particular reagents are mixed with a blood sample to facilitate performance of each of the assays. Each assay is generally described in the next paragraphs.

The INTEM assay is the typical screening test for coagulation disorders. The clotting time (CT) gives information on the clotting factor activity(s) of the intrinsic and common pathway or on presence of heparin in the sample. The clot formation time (CFT) gives information on the speed of clot formation which is mainly influenced by the thrombin generation, platelets and fibrinogen levels. Similar information to CFT is also given by the alpha angle. The clot firmness parameters (e.g., A5, A10, MCF) give information on the overall clot firmness, which is a function for platelet activity, fibrinogen concentration and F XIII (factor XIII) availability. Deviations of the parameters from the established reference ranges indicate a potential coagulation disturbance.

The HEPTEM assay provides information that is similar to the INTEM assay but eliminates a potential heparin effect. HEPTEM is useful for cardiac surgery where patients are highly heparinized and coagulation tests may become less meaningful because of the heparin effect in the sample. In the HEPTEM assay, the heparin is digested rapidly and the resulting ROTEM® result reflects the coagulation status of the patient in the absence of heparin. HEPTEM helps to rationally treat a heparinized patient under transient high dose heparin therapy and to identify a heparin effect in combination with the INTEM assay.

The EXTEM assay is a screening test for coagulation disorders. The CT gives information on the clotting factor activity(s) of the extrinsic or common pathways or on the presence of oral anticoagulants, e.g. vitamin K antagonists or direct thrombin inhibitors. The CFT gives information on the speed of clot formation which is substantially influenced by the thrombin generation, platelets and fibrinogen levels. Similar information to CFT is also given by the alpha angle. The clot firmness parameters (e.g., A5, A10, MCF) give information on the overall clot firmness, which is related to platelet activity, fibrinogen concentration and F XIII availability. Deviations of the parameters from the established reference ranges indicate a potential coagulation disturbance.

Using the FIBTEM assay, the quality of fibrin polymerization or the fibrinogen level can be estimated quickly. The clot firmness parameters give information on the overall clot firmness.

The APTEM assay provides information regarding coagulation without fibrinolysis effects. Fibrinolytic processes are detected by a loss of the clot firmness during the clot formation analysis with the ROTEM® in the EXTEM assay.

In some embodiments, the thromboelastometry analyzer console 110 may be used to perform some or all of the assays described above. Alternatively, or additionally, in some embodiments the second thromboelastometry analyzer console 130 may be used to perform some or all of the assays described above, and the results from the second thromboelastometry analyzer console 130 may be communicated via the data communication network 102 to the thromboelastometry analyzer console 110 for additional analysis.

In step 230, the thromboelastometry analyzer console 110 optionally receives data from one or more external analytical testing devices. For example, in the context of blood testing system 100 the thromboelastometry analyzer console 110 may optionally receive data via the data communication network 102 from one or more of the second thromboelastometry analyzer console 130, the blood gas analyzer 140, and/or the platelet aggregation analyzer 150. The thromboelastometry analyzer console 110 can thereby beneficially receive data pertaining to the patient that is not directly attainable using the analyzer console 110 alone. For example, the blood gas analyzer 140 may provide to the thromboelastometry analyzer console 110 information about the patient such as, but not limited to, hemoglobin concentration (Hb), base excess (BE), pH of the blood, lactate, Cai²⁺, partial pressures of carbon dioxide and oxygen, bicarbonate level, and the like. In addition, in some embodiments the platelet aggregation analyzer 150 can provide measurements pertaining to platelet aggregation of the patient's blood. In one nonlimiting example, a ROTEM® *platelet* system (available from Tem International GmbH) performs at least two of three different assays for assessing platelet function: ARATEM, ADPTEM, and TRAPTEM. The ARATEM assay can be used, for example, for the detection of cyclooxygenase inhibitors. The ADPTEM assay can be used, for example for the detection of adenosine diphosphate (ADP) receptor blockage. The TRAPTEM assay can be used, for example, for the detection of glycoprotein (GP) IIb/IIIa or PAR-1 (protease-activated receptor-1) receptor antagonists.

In step 240, algorithm-specific threshold values are accessed by the thromboelastometry analyzer console 110. In some embodiments, the algorithm-specific threshold values are stored in memory of the thromboelastometry analyzer console 110. Alternately, or additionally, in some embodiments the algorithm-specific threshold values are stored in another database that is accessible by the thromboelastometry analyzer console 110, such as using the data communication network 102.

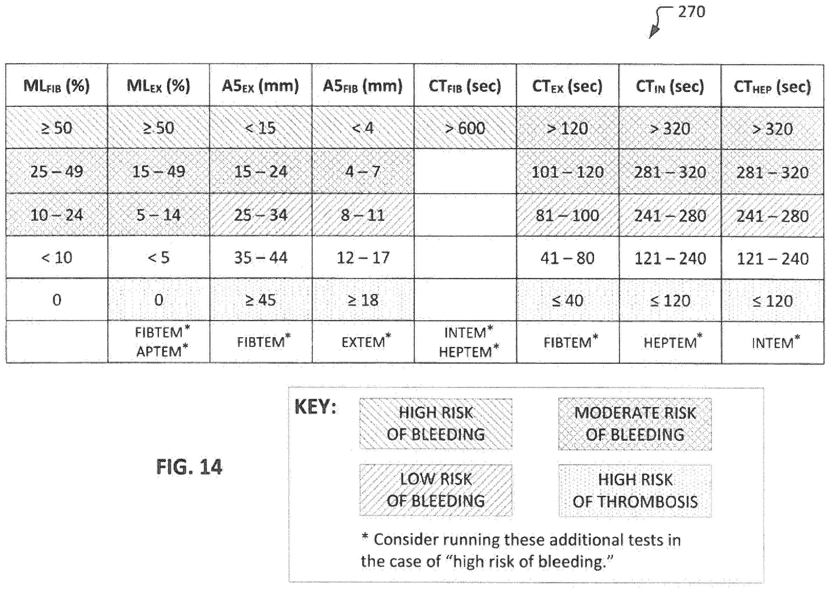

An example of algorithm-specific threshold values is shown in risk matrix 270 of FIG. 14. These threshold values are referred to herein as "algorithm-specific" because, as described further below, different algorithms are available (e.g., trauma, cardiovascular surgery, liver surgery or GI bleeding, PPH, and the like) and each algorithm can have a unique set of threshold values established. The risk matrix 270 illustrates some algorithm-specific threshold values that may be appropriate for a trauma algorithm, for example. As described further below, the algorithm-specific threshold values (from risk matrix 270 for example) are usable within an algorithm that can result in recommendations for patient treatment, further testing, and the like. Alternatively, the threshold values can be organized in any other suitable format other than a table.

In the example of risk matrix 270, threshold values for particular parameters (e.g., "ML_{FIB}," "ML_{EX}," "A5_{EX}," etc.) are established. For example, as described above, ML_{FIB} quantifies the maximum lysis as measured using a FIBTEM assay; ML_{EX} quantifies the maximum lysis as measured using an EXTEM assay; and A5_{EX} quantifies the amplitude (clot firmness) 5 minutes after CT in the EXTEM assay. As depicted in example risk matrix 270, a ML_{FIB} value of ≥ 50% can be established such that it is associated with a high risk of bleeding. Likewise, an A5_{EX} value in the range of 15-24 mm can be associated with a moderate risk of bleeding.

In some implementations, color coding of information such as the threshold values and the algorithm results and/or recommendations presented by the thromboelastometry analyzer console 110 is beneficially used. For example, a clinician user of the thromboelastometry analyzer console 110 may be readily able to ascertain a high risk situation when information is presented in a red color, in contrast, for example to an orange or yellow color which may be defined to represent lower risks. Such color coding is described further below.

Risk matrix 270 also illustrates how particular messages with recommendations can be established for additional testing when certain criteria are met. For example, as depicted in risk matrix 270 a FIBTEM assay and an APTEM assay will be recommended in response to a ML_{EX} value that is ≥ 50% (which represents a high risk of bleeding in this example).

The threshold values and recommendations for additional testing (as defined in the risk matrix 270, for example) can be established and customized by a particular local institution (or by a particular doctor, if so desired) in keeping with the preferences of the particular local institution (or particular doctor). This customization allows each local institution to establish threshold values and recommendations for additional testing that are based on and/or consistent with the institution's specific approach to clinical analysis and patient care. In addition, the establishment of such threshold values and recommendations for additional testing facilitates standardization of clinical analysis and patient care within a local institution, or potentially across multiple institutions if the multiple institutions desire to so coordinate.

Still referring to FIG. 2, in step 250 the thromboelastometry analyzer console 110 applies a user-selected algorithm to processes thromboelastometry data, data received from one or more external analytical testing devices (optionally), and the algorithm-specific threshold values. The outcome of running the algorithm is the potential determination of recommendations for patient treatment and/or further testing. As a threshold matter, before using any data received from the one or more external analytical testing devices in the algorithm, the thromboelastometry analyzer console 110 will verify that the data pertains to the appropriate patient and is current (e.g., measure within 30 minutes of the thromboelastometry measurements).

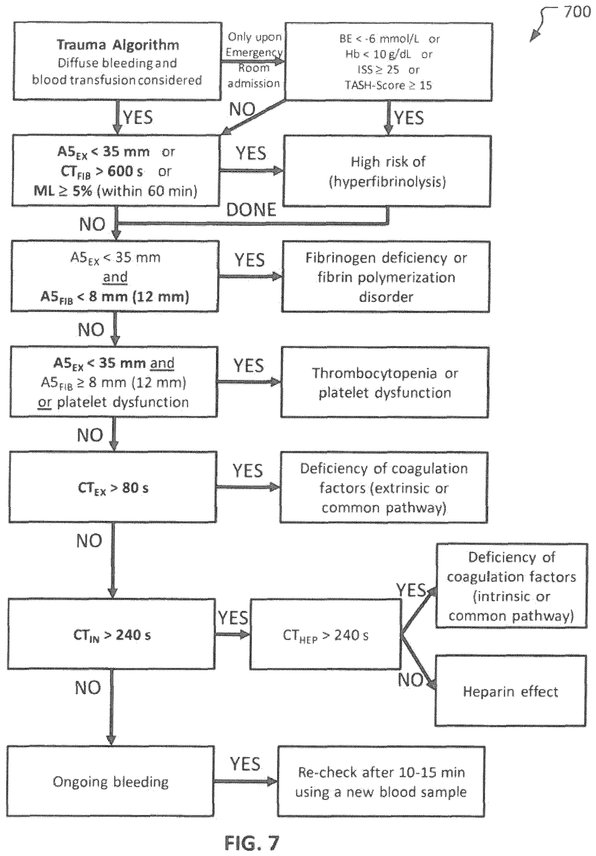

Examples of algorithms are provided and described below in reference to FIGS. 7-11. In some embodiments, the thromboelastometry analyzer console 110 informs the user if parameters or assays are missing for adequate data interpretation according to the diagnostic algorithm being run.

In step 260, results are displayed by the thromboelastometry analyzer console 110. For example, in some embodiments the results are displayed on the touchscreen display 120 of the thromboelastometry analyzer console 110. At least two different types of results presentations are envisioned: thromboelastometry results 262 and messages that may include recommendations (based on algorithm results) 264. In some embodiments, the user can selectively switch ("toggle") between the displays of different modes of results presentations, (e.g., from displaying the one or more messages based on algorithm results to displaying graphical or numeric information corresponding to at least some of the blood test result data).

An example of displaying thromboelastometry results 262 is depicted in FIG. 13. Such thromboelastometry results 262 include one or more graphics that are continuous plots of the firmness of a blood clot during the performance of an assay, as depicted in FIG. 12, for example.

An example of displaying recommendations (based on algorithm results) 264 is depicted in the screen shot 121 of FIG. 1. In this example, the displayed message includes a recommendation for the attending physician to consider: "Consider: 1. Fibrinogen deficiency or fibrin polymerization disorder."

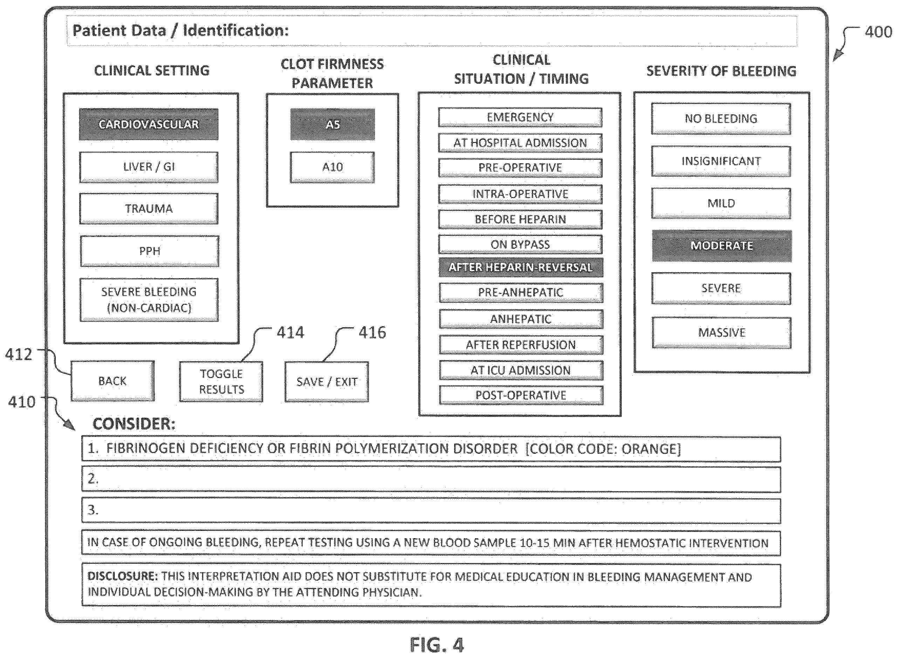

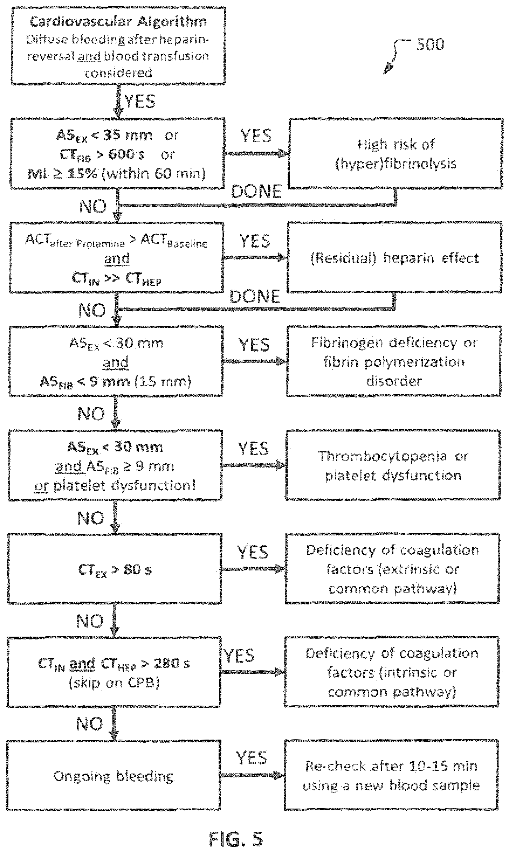

Referring to **FIGS. 4** **and** **5**, an example performance of a cardiovascular algorithm is depicted. FIG. 4 illustrates a user interface 400 of a display device, such as the user interface display 120 of the thromboelastometry analyzer console 110 (refer to FIG. 1). FIG. 5 illustrates an example cardiovascular algorithm 500 that can be run by the thromboelastometry analyzer console 110 (e.g., as in step 250 of process 200 depicted in FIG. 2). The cardiovascular algorithm 500 includes parameter threshold values that can be established by a user (e.g., refer to risk matrix 270 of FIG. 14).

In this illustrative example, the following information is assumed: a clinical setting of "Cardiovascular" is selected, a clot firmness parameter of "A5" is selected, a clinical situation/timing of "After Heparin-Reversal" is selected, and a severity of bleeding of "Moderate" is selected. In addition, the following example test parameter results are assumed (solely for the purpose of illustrating how the algorithm 500 can be applied-because such test parameter results can vary): the CT_{EX} is 75 seconds, the CT_{IN} is 210 seconds, the CT_{HEP} is 205 seconds, the A5_{EX} is 22 mm, the A5_{FIB} is 4 mm, the ML_{EX} is 7%, and the ACT is 120 seconds (as determined by system for measuring activated coagulation time).

Applying the information and test parameters to the example cardiovascular algorithm 500, the resulting recommendation is to consider "Fibrinogen deficiency or fibrin polymerization disorder" as shown in the results 410 of user interface 400. In this example, the recommendation is color coded in orange to indicate that the recommendation is important (e.g., a moderate risk of bleeding may exist), but not as critical as if the recommendation were to be color coded in red. Such a color coding determination can be made as a result from the application of threshold values from a risk matrix (e.g., refer to risk matrix 270 of FIG. 14).

The aforementioned resulting recommendation is determined by applying the example cardiovascular algorithm 500 as follows. Because ML is less than 15%, the first action step is answered in the negative. Next, because ACT is normal and CT_{IN} is similar to CT_{HEP}, the second action step is also answered in the negative. Then, because A5_{EX} (which is given as 22 mm) is less than 30 mm, and A5_{FIB} (which is given as 4 mm) is less than 9 mm, the third action step is answered in the positive. Therefore, the resulting recommendation is to consider "Fibrinogen deficiency or fibrin polymerization disorder."

As described above, the threshold values and recommendations for further testing, as defined in a risk matrix, can be customized by a local institution. In addition, the algorithms can be customized by a local institution. For example, some steps from the example cardiovascular algorithm 500 can be omitted, and/or other steps can be added. In one such example, the step to identify hyperfibrinolysis may be chosen to be omitted in a hospital where anti-fibrinolytic drugs are administered routinely (prophylactically).

Other user input options can also be provided, as illustrated in the example user interface 400. For example, selection of a "Back" button 412 can allow the user to step the user interface 400 backward to re-enter data, for example. Selection of a "Toggle Results" button 414 can allow the user to switch between displaying the user interface 400 and a screen that shows one or more graphics that are continuous plots of the firmness of a blood clot during the performance of an assay (e.g., as depicted in FIG. 12). Selection of a "Save/Exit" button 416 can allow the user to save the results and exit the session that is running the cardiovascular algorithm 500.

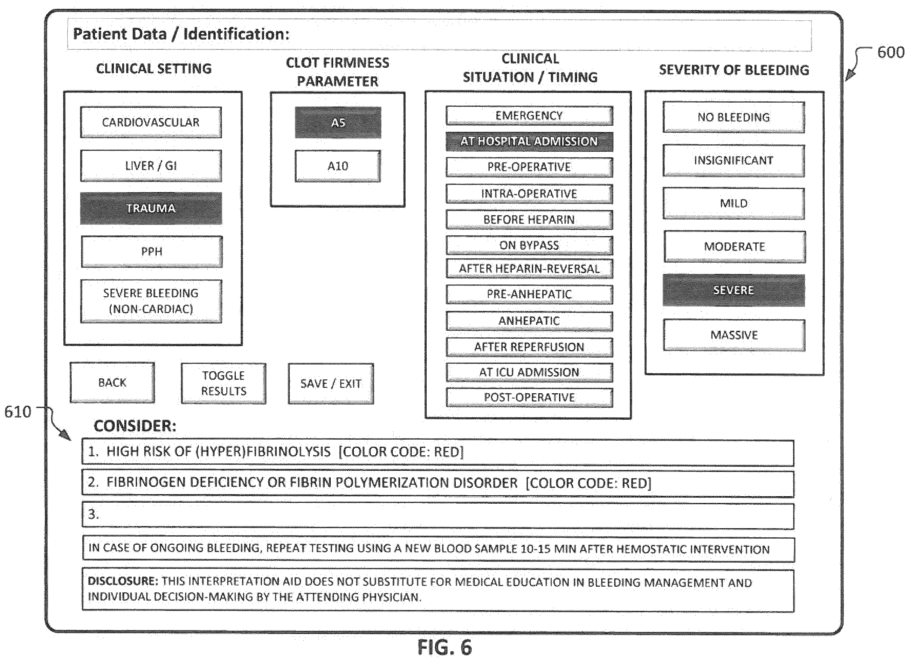

Referring to **FIGS. 6** **and** **7**, an example performance of a trauma algorithm is depicted. FIG. 6 illustrates a user interface 600 of a display device, such as the user interface display 120 of the thromboelastometry analyzer console 110 (refer to FIG. 1). FIG. 7 illustrates an example cardiovascular algorithm 700 that can be run by the thromboelastometry analyzer console 110 (e.g., as in step 250 of process 200 depicted in FIG. 2).

In this example, the following information is assumed: a clinical setting of "Trauma" is selected, a clot firmness parameter of "A5" is selected, a clinical situation/timing of "At Hospital Admission" is selected, and a severity of bleeding of "Severe" is selected. In addition, the following test parameter results are assumed: the CT_{EX} is 85 seconds, the CT_{IN} is 190 seconds, the CT_{HEP} is 180 seconds, the A5_{EX} is 14 mm, the A5_{FIB} is 2 mm, the ML_{EX} is 6%, the Hb is 7 g/dL (as determined by a BGA), the pH is 7.1 (as determined by a BGA), and the BE is -8 mmol/L (as determined by a BGA).

Applying the information and test parameters to the example cardiovascular algorithm 700, the resulting recommendations are to consider "High risk of (hyper)fibrinolysis" and "Fibrinogen deficiency or fibrin polymerization disorder" as shown in the results 610 of user interface 600. In this example, the "High risk of (hyper)fibrinolysis" and the "Fibrinogen deficiency or fibrin polymerization disorder" are both color coded in red to indicate a high risk. Such a color coding determination can be made as a result from the application of threshold values from a risk matrix (e.g., refer to risk matrix 270 of FIG. 14).

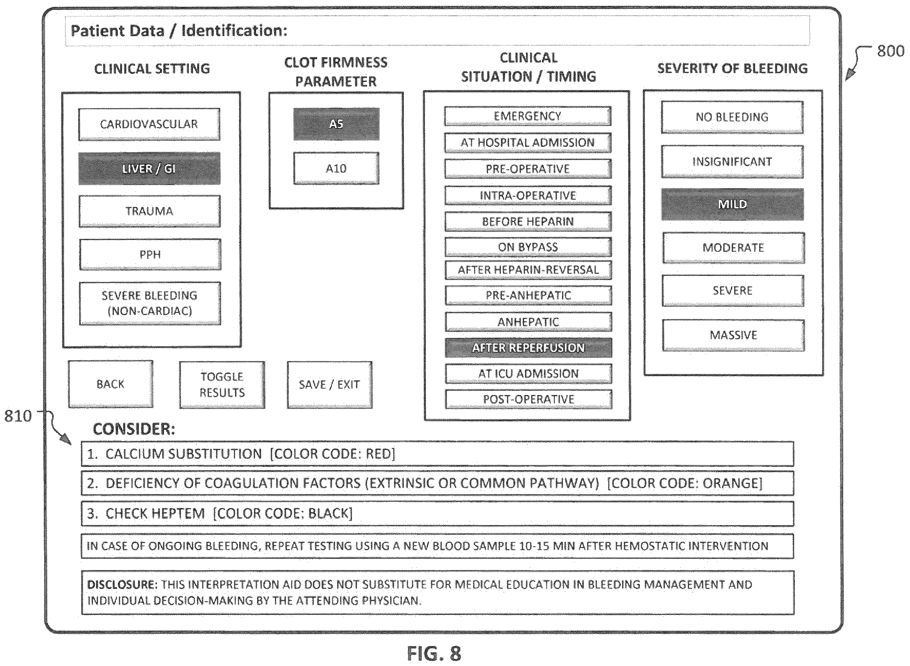

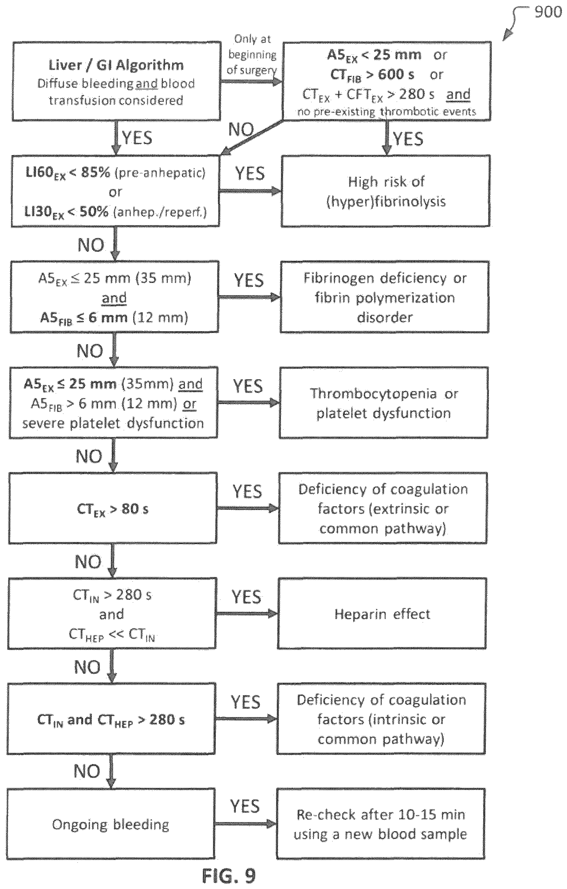

Referring to **FIGS. 8** **and** **9**, an example performance of a liver surgery/gastrointestinal (GI) bleeding algorithm is depicted. FIG. 8 illustrates a user interface 800 of a display device, such as the user interface display 120 of the thromboelastometry analyzer console 110 (refer to FIG. 1). FIG. 9 illustrates an example liver surgery/gastrointestinal (GI) bleeding algorithm 900 that can be run by the thromboelastometry analyzer console 110 (e.g., as in step 250 of process 200 depicted in FIG. 2).

In this example, the following information is assumed: a clinical setting of "Liver/GI" is selected, a clot firmness parameter of "A5" is selected, a clinical situation/timing of "After Reperfusion" is selected, and a severity of bleeding of "Mild" is selected. In addition, the following test parameter results are assumed: the CT_{EX} is 110 seconds, the CT_{IN} is 260 seconds, the A5_{EX} is 37 mm, the A5_{FIB} is 12 mm, the ML_{EX} is 8%, the Hb is 9 g/dL (as determined by a BGA), the pH is 7.3 (as determined by a BGA), the BE is -2 mmol/L (as determined by a BGA), and the Cai²⁺ is 0.8 mmol/L (as determined by a BGA).

Applying the information and test parameters to the example cardiovascular algorithm 900, the resulting recommendations are to consider "Calcium Substitution," "Deficiency of Coagulation Factors (Extrinsic or Common Pathway)," and "Check HEPTEM" as shown in the results 810 of user interface 800. In this example, the "Calcium Substitution" is color coded in red, the "Deficiency of Coagulation Factors (Extrinsic or Common Pathway)" is color coded in orange, and the "Check HEPTEM" is color coded in black. The color coding represents the risk or urgency of the issues leading to the recommendations. Such color coding determinations can be made as a result from the application of threshold values from a risk matrix (e.g., refer to risk matrix 270 of FIG. 14).

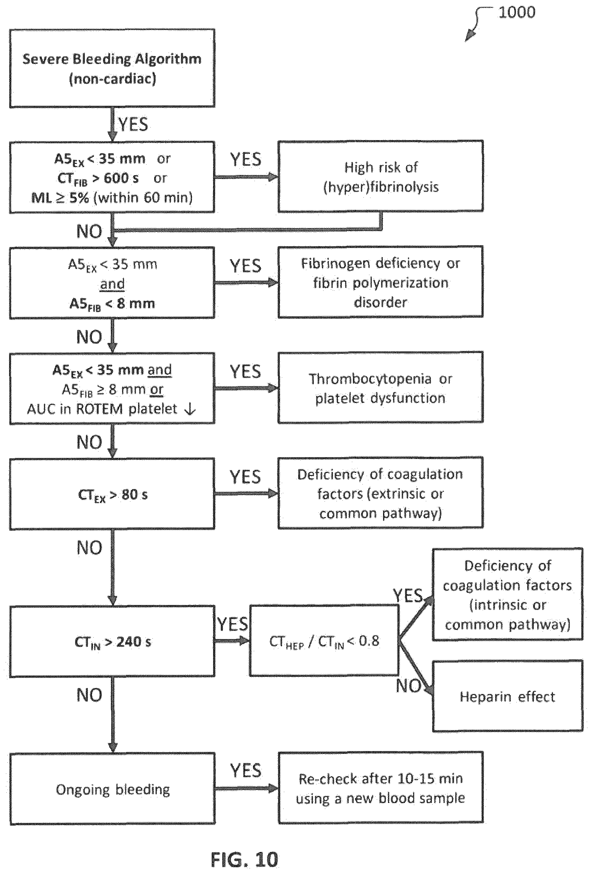

Referring to **FIG. 10**, an example severe bleeding (non-cardiac) algorithm 1000 is provided. As with the examples provided above, the severe bleeding (non-cardiac) algorithm 1000 can be applied in the context of particular clinical information and test parameter results to arrive at recommendations that are presented to the user. The severe bleeding (non-cardiac) algorithm 1000 includes parameter threshold values that can be established by a user (e.g., refer to risk matrix 270 of FIG. 14). In addition, the severe bleeding (non-cardiac) algorithm 1000 can be customized by a local institution. For example, some steps from the example severe bleeding (non-cardiac) algorithm 1000 can be omitted, revised, and/or added.

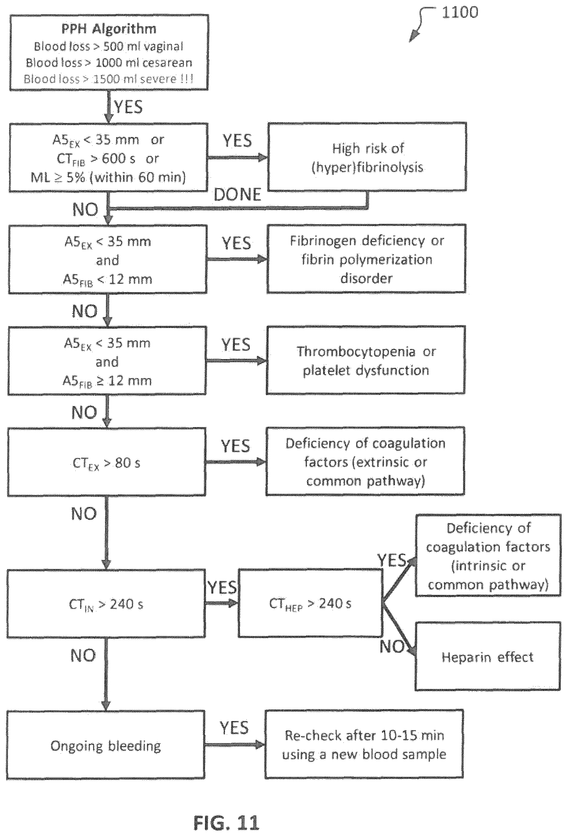

Referring to **FIG. 11**, an example PPH algorithm 1100 is provided. As with the examples provided above, the PPH algorithm 1100 can be applied in the context of particular clinical information and test parameter results to arrive at recommendations that are presented to the user. The PPH algorithm 1100 includes parameter threshold values that can be established by a user (e.g., refer to risk matrix 270 of FIG. 14). In addition, the PPH algorithm 1100 can be customized by a local institution. For example, some steps from the example PPH algorithm 1100 can be omitted, revised, and/or added.

A number of embodiments of the invention have been described. Nevertheless, it will be understood that various modifications may be made without departing from the scope of the invention as defined by the appended claims.

## Claims

1. A method of providing result interpretation output, comprising:
receiving, at a blood analyzer machine (110), user input indicative of a clinical setting of a patient that provided a blood sample;
receiving, at the blood analyzer machine (110), the blood sample of the patient;
performing, by the blood analyzer machine (110), one or more blood characteristic tests on the blood sample for providing blood test result data;
interpreting, by the blood analyzer machine (110) and using an algorithm from a plurality of algorithms accessible to the blood analyzer machine (110), the blood test result data provided from the one or more blood characteristic tests; and
displaying, using a user interface (120) of the blood analyzer machine (110), one or more messages indicative of a potential diagnosis or health condition based on said interpreting the blood test result data provided from the one or more blood characteristic tests;
wherein the blood analyzer machine (110) selects a particular algorithm of a plurality of the algorithms for interpreting the blood test result data in response to the user input indicative of the clinical setting of the patient; and
wherein said interpreting by the blood analyzer machine (110) comprises analyzing the blood test result data in comparison to one or more preset algorithm-specific threshold values provided in a risk matrix (270) using the algorithm so as to determine said one or more messages,
further comprising:
receiving at the blood analyzer machine (110), via one or more data cables or via a wireless network connection (102) for transferring data to the blood analyzer machine, external test result data from one or more external blood analyzer devices (130, 140, 150) that are different from and remote from said blood analyzer machine (110), said one or more external blood analyzer devices (130, 140 , 150) being connected to the blood analyzer machine (110), the external test result data from the one or more external blood analyzer devices (130, 140, 150) resulting from additional blood characteristic tests on the blood sample by the one or more external blood analyzer devices (130, 140, 150);
wherein said interpreting by the blood analyzer machine (110) includes analyzing, using said algorithm, the blood test result data from the blood analyzer machine and the external test result data from the one or more external blood analyzer devices (130, 140, 150), wherein the one or more messages displayed by the blood analyzer machine (110) are based on said interpreting the blood test result data from the blood analyzer machine (110) and the external test result data from the one or more external blood analyzer devices (130, 140, 150),
the interpreting further comprising comparing and contrasting the test results with each other using a computerized algorithm.

2. The method of claim 1,
wherein the one or more messages displayed by the blood analyzer machine (110) are a result interpretation output that is different from blood test result data output from the blood analyzer machine.

3. The method of claim 1,
wherein the performing of the one or more blood characteristic tests by the blood analyzer machine (110) on the blood sample includes measuring blood coagulation characteristics.

4. The method of claim 1, further comprising:
after displaying the one or more messages, receiving, at the blood analyzer machine (110), a user selection of a selectable element for changing a display mode of the user interface (120); and
in response to receiving the user selection, changing from displaying (120) the one or more messages to displaying graphical or numeric information corresponding to at least some of the blood test result data.

5. The method of claim 1,
wherein the one or more preset threshold values are customizable by an operator of the blood analyzer machine (110) so as to modify one or more default threshold values to one or more customized threshold values.

6. The method of claim 1 or 5,
wherein the blood analyzer machine (110) comprises a control console including a user interface display device (120), one or more computer processors, and a computer-readable memory device, wherein the plurality of algorithms accessible to the blood analyzer machine (110) and the one or more preset threshold values are stored by the computer-readable memory device.

7. Blood analyzer system (100) for providing result interpretation output, comprising:
a blood analyzer machine (110) configured to generate blood test result data by performing one or more blood characteristic tests;
a user interface (120) comprising a display device configured to display the blood test result data;
one or more processors;
a computer memory device storing computer-readable instructions for one or more algorithms for interpreting the blood test result data, wherein the computer-readable instructions, when executed by the one or more processors, are configured to cause the system (100) to perform at least the following operations:
analyze the blood test result data; and
output via the user interface (120) one or more messages indicative of a potential diagnosis or health condition based on the analysis of the blood test result data;
wherein the computer-readable instructions, when executed by the one or more processors, are configured to cause the system (100) to prompt a user for input indicative of a clinical setting of a patient; and
the computer-readable instructions, when executed by the one or more processors, are configured to cause the system (100) to select a particular algorithm of a plurality of the algorithms for interpreting the blood test result data in response to the user input indicative of the clinical setting of the patient;
**characterized in that** said analyzing of the blood test result data comprises analyzing the blood test result data in comparison to one or more preset algorithm-specific threshold values provided in a risk matrix (270) using the algorithm so as to output said one or more messages;
wherein the computer-readable instructions, when executed by the one or more processors, are configured to cause the system (100) to receive external test result data from one or more external blood analyzer devices (130, 140, 150) that are different from and remote from said blood analyzer system (100), wherein the blood analyzer system (100) comprises a data connection for receiving the external test result data from the one or more external blood analyzer devices (130, 140, 150), the data connection comprising a data cable connection or a wireless network connection (102); and
wherein said interpreting by the blood analyzer machine (110) includes analyzing, using said algorithm, the blood test result data from the blood analyzer machine and the external test result data from the one or more external blood analyzer devices (130, 140, 150), wherein the one or more messages displayed by the blood analyzer machine (110) are based on said interpreting the blood test result data from the blood analyzer machine (110) and the external test result data from the one or more external blood analyzer devices (130, 140, 150),
the interpreting further comprising comparing and contrasting the test results with each other using a computerized algorithm.

8. The system (100) of claim 7,
wherein the blood testing device (110) is a thromboelastometry testing device (110) including a movable probe to interact with at least a portion of a blood sample for measuring blood coagulation characteristics of the blood sample; and
wherein the blood test result data are indicative of the blood coagulation characteristics.

9. The system (100) of claim 7 or 8,
wherein the one or more messages indicative of a potential diagnosis or health condition are a result interpretation output that is displayed by the display device and that is different from blood test result data.

## Patentansprüche

1. Verfahren zum Bereitstellen einer Ergebnisinterpretationsausgabe, umfassend:
Empfangen einer Benutzereingabe an einem Blutanalysegerät (110), die eine klinische Einstellung eines Patienten anzeigt, der eine Blutprobe bereitgestellt hat;
Empfangen der Blutprobe des Patienten an dem Blutanalysegerät (110);
Durchführen eines oder mehrerer Bluteigenschaftstests an der Blutprobe durch das Blutanalysegerät (110), um Bluttestergebnisdaten bereitzustellen;
Interpretieren der aus dem einen oder den mehreren Bluteigenschaftstests bereitgestellten Blutergebnisdaten durch das Blutanalysegerät (110) und Verwenden eines Algorithmus aus mehreren Algorithmen, auf die das Blutanalysegerät (110) zugreifen kann; und
Anzeigen unter Verwendung einer Benutzerschnittstelle (120) des Blutanalysegeräts (110) einer oder mehrerer Nachrichten, die eine mögliche Diagnose oder einen möglichen Gesundheitszustand anzeigen, auf der Grundlage der Interpretation der aus dem einen oder den mehreren Bluteigenschaftstests bereitgestellten Blutergebnisdaten;
wobei das Blutanalysegerät (110) einen bestimmten Algorithmus aus einer Vielzahl von Algorithmen zum Interpretieren der Bluttestergebnisdaten in Reaktion auf die Benutzereingabe auswählt, welche die klinische Einstellung des Patienten anzeigt; und
wobei das Interpretieren durch das Blutanalysegerät (110) das Analysieren der Bluttestergebnisdaten im Vergleich zu einem oder mehreren voreingestellten algorithmenspezifischen Schwellenwerten, die in einer Risikomatrix (270) bereitgestellt werden, unter Verwendung des Algorithmus umfasst, um die eine oder die mehreren Nachrichten zu bestimmen,
ferner umfassend:
Empfangen von externen Testergebnisdaten von einem oder mehreren externen Blutanalysegeräten (130, 140, 150) an dem Blutanalysegerät (110) über ein oder mehrere Datenkabel oder über eine drahtlose Netzwerkverbindung (102) zum Übertragen von Daten an das Blutanalysegerät, die sich von dem Blutanalysegerät (110) unterscheiden und von diesem entfernt sind, wobei das eine oder die mehreren externen Blutanalysegeräte (130, 140, 150) mit dem Blutanalysegerät (110) verbunden sind, wobei die externen Testergebnisdaten von dem einen oder mehreren externen Blutanalysegeräte (130, 140, 150) aus zusätzlichen Bluteigenschaftstests an der Blutprobe durch das eine oder die mehreren externen Blutanalysegeräte (130, 140, 150) resultieren;
wobei das Interpretieren durch das Blutanalysegerät (110) das Analysieren der Bluttestergebnisdaten von dem Blutanalysegerät und der externen Testergebnisdaten von der einen oder den mehreren externen Blutanalysegerätvorrichtungen (130, 140, 150) unter Verwendung des Algorithmus umfasst, wobei die eine oder die mehreren von dem Blutanalysegerät (110) angezeigten Nachrichten auf dem Interpretieren der Bluttestergebnisdaten von dem Blutanalysegerät (110) und der externen Testergebnisdaten von dem einen oder den mehreren externen Blutanalysegerätgeräten (130, 140, 150) basieren,
wobei das Interpretieren ferner das Vergleichen und Gegenüberstellen der Testergebnisse unter Verwendung eines computergestützten Algorithmus umfasst.

2. Verfahren nach Anspruch 1,
wobei die eine oder mehreren von dem Blutanalysegerät (110) angezeigten Nachrichten eine Ergebnisinterpretationsausgabe sind, die sich von den von dem Blutanalysegerät ausgegebenen Bluttestergebnisdaten unterscheidet.

3. Verfahren nach Anspruch 1,
wobei das Durchführen eines oder mehrerer Bluteigenschaftstests durch das Blutanalysegerät (110) an der Blutprobe das Messen von Blutgerinnungseigenschaften umfasst.

4. Verfahren nach Anspruch 1, ferner umfassend:
nach dem Anzeigen der einen oder mehreren Nachrichten, Empfangen einer Benutzerauswahl eines auswählbaren Elements zum Ändern eines Anzeigemodus der Benutzerschnittstelle (120) an dem Blutanalysegerät (110); und
als Reaktion auf das Empfangen der Benutzerauswahl, Ändern von der Anzeige (120) der einen oder mehreren Nachrichten zur Anzeige von grafischen oder numerischen Informationen, die mindestens einigen der Bluttestergebnisdaten entsprechen.

5. Verfahren nach Anspruch 1,
wobei der eine oder die mehreren voreingestellten Schwellenwerte durch einen Bediener des Blutanalysegeräts (110) anpassbar sind, um einen oder mehrere Standardschwellenwerte in einen oder mehrere angepasste Schwellenwerte umzuwandeln.

6. Verfahren nach Anspruch 1 oder 5,
wobei das Blutanalysegerät (110) eine Steuerkonsole mit einer Benutzerschnittstellenanzeigevorrichtung (120), einem oder mehreren Computerprozessoren und einer computerlesbaren Speichervorrichtung umfasst, wobei die Vielzahl von Algorithmen für das Blutanalysegerät (110) zugänglich ist und der eine oder die mehreren voreingestellten Schwellenwerte von der computerlesbaren Speichervorrichtung gespeichert werden.

7. Blutanalysesystem (100) zum Bereitstellen einer Ergebnisinterpretationsausgabe, umfassend:
eine Blutanalysemaschine (110), die ausgebildet ist, um Bluttestergebnisdaten durch Ausführen eines oder mehrerer Bluteigenschaftstests zu erzeugen;
eine Benutzerschnittstelle (120) mit einer Anzeigevorrichtung, die zum Anzeigen der Blutuntersuchungsergebnisdaten ausgebildet ist;
ein oder mehrere Prozessoren;
eine Computerspeichervorrichtung, die computerlesbare Anweisungen für einen oder mehrere Algorithmen zum Interpretieren der Bluttestergebnisdaten speichert, wobei die computerlesbaren Anweisungen, wenn sie von dem einen oder den mehreren Prozessoren ausgeführt werden, so ausgebildet sind, dass sie das System (100) veranlasst,
mindestens die folgenden Operationen durchzuführen:
Analysieren der Blutuntersuchungsergebnisdaten; und
Ausgabe einer oder mehrerer Nachrichten über die Benutzerschnittstelle (120), die eine mögliche Diagnose oder einen möglichen Gesundheitszustand auf der Grundlage der Analyse der Bluttestergebnisdaten anzeigen;
wobei die computerlesbaren Anweisungen, wenn sie von dem einen oder den mehreren Prozessoren ausgeführt werden, ausgebildet sind, um das System (100) zu veranlassen, einen Benutzer zu einer Eingabe aufzufordern, die eine klinische Einstellung eines Patienten anzeigt; und
die computerlesbaren Anweisungen, wenn sie von dem einen oder den mehreren Prozessoren ausgeführt werden, so ausgebildet sind, dass sie das System (100) veranlassen, einen bestimmten Algorithmus aus einer Vielzahl von Algorithmen zum Interpretieren der Bluttestergebnisdaten in Reaktion auf die Benutzereingabe auszuwählen, die das klinische Umfeld des Patienten anzeigt;
**dadurch gekennzeichnet, dass**
das Analysieren der Bluttestergebnisdaten das Analysieren der Bluttestergebnisdaten im Vergleich zu einem oder mehreren voreingestellten algorithmusspezifischen Schwellenwerten, die in einer Risikomatrix (270) bereitgestellt werden, unter Verwendung des Algorithmus umfasst, um die eine oder mehreren Nachrichten auszugeben;
wobei die computerlesbaren Anweisungen, wenn sie von dem einen oder den mehreren Prozessoren ausgeführt werden, ausgebildet sind, um zu bewirken, dass das System (100) externe Testergebnisdaten von einem oder mehreren externen Blutanalysegeräten (130, 140, 150) empfängt, die sich von von dem Blutanalysesystem (100) unterscheiden und davon entfernt sind, wobei das Blutanalysesystem (100) eine Datenverbindung zum Empfangen der externen Testergebnisdaten von der einen oder den mehreren externen Blutanalysegeräten (130, 140, 150) umfasst, wobei die Datenverbindung eine Datenkabelverbindung oder eine drahtlose Netzwerkverbindung (102) umfasst; und
wobei das Interpretieren durch das Blutanalysegerät (110) das Analysieren der Bluttestergebnisdaten von dem Blutanalysegerät und der externen Testergebnisdaten von der einen oder den mehreren externen Blutanalysegerätvorrichtungen (130, 140, 150) unter Verwendung des Algorithmus umfasst, wobei die eine oder die mehreren von dem Blutanalysegerät (110) angezeigten Nachrichten auf dem Interpretieren der Bluttestergebnisdaten von dem Blutanalysegerät (110) und der externen Testergebnisdaten von dem einen oder den mehreren externen Blutanalysegerätgeräten (130, 140, 150) basieren,
wobei das Interpretieren ferner das Vergleichen und Gegenüberstellen der Testergebnisse unter Verwendung eines computergestützten Algorithmus umfasst.

8. System (100) nach Anspruch 7,
wobei die Bluttestvorrichtung (110) eine Thromboelastometrietestvorrichtung (110) ist, die eine bewegliche Sonde umfasst, um mit mindestens einem Teil einer Blutprobe zu interagieren, um die Blutgerinnungseigenschaften der Blutprobe zu messen; und wobei die Bluttestergebnisdaten die Blutgerinnungseigenschaften anzeigen.

9. System (100) nach Anspruch 7 oder 8,
wobei die eine oder die mehreren Meldungen, die eine mögliche Diagnose oder einen möglichen Gesundheitszustand anzeigen, eine Ergebnisinterpretationsausgabe sind, die von der Anzeigevorrichtung angezeigt wird und die sich von den Bluttestergebnisdaten unterscheidet.

## Revendications

1. Procédé de fourniture d'une sortie d'interprétation de résultats, comprenant :
la réception, à une machine d'analyseur de sang (110), d'une entrée d'utilisateur indicatrice d'un paramètre clinique d'un patient qui a fourni un échantillon de sang ;
la réception, à la machine d'analyseur de sang (110), de l'échantillon du sang du patient ;
la réalisation, par la machine d'analyseur de sang (110), d'un ou de plusieurs tests caractéristiques du sang sur l'échantillon de sang pour fournir des données de résultats de test de sang ;
l'interprétation, par la machine d'analyseur de sang (110) et en utilisant un algorithme d'une pluralité d'algorithmes accessibles à la machine d'analyseur de sang (110), des données de résultats de test de sang fournies à partir d'un ou de plusieurs tests caractéristiques du sang ; et
l'affichage, en utilisant une interface d'utilisateur (120) de la machine d'analyseur de sang (110), d'un ou de plusieurs messages indicateurs d'un diagnostic potentiel ou d'un état sanitaire basé sur ladite interprétation des données de résultats de test du sang fournies à partir de l'un ou de plusieurs tests caractéristiques du sang ;
dans lequel la machine d'analyseur de sang (110) sélectionne un algorithme particulier d'une pluralité d'algorithmes pour interpréter les données de résultats de test du sang en réponse à l'entrée de l'utilisateur indicatrice du paramètre clinique du patient ; et
dans lequel ladite interprétation par la machine d'analyseur de sang (110) comprend l'analyse des des données de résultats de test du sang en comparaison à une ou plusieurs valeurs de seuil spécifiques d'un algorithme préréglé fournies dans une matrice de risque (270) en utilisant l'algorithme de manière à déterminer lesdits un ou plusieurs messages,
comprenant en outre :
la réception à la machine d'analyseur de sang (110), via un ou plusieurs câbles de données ou via une connexion de réseau sans fil (102) pour transférer les données à la machine d'analyseur de sang, des données de résultats de test externe d'un ou de plusieurs dispositifs d'analyseur de sang externes (130, 140, 150) qui sont différents de et à distance de ladite machine d'analyseur de sang (110), lesdits un ou plusieurs dispositifs d'analyseur de sang externes (130, 140, 150) étant connectés à la machine d'analyseur de sang (110), les données de résultats de test externe de l'un ou de plusieurs dispositifs d'analyseur de sang externe (130, 140, 150) résultant de tests caractéristiques du sang supplémentaires sur l'échantillon de sang par les un ou plusieurs dispositifs d'analyseur de sang externes (13, 140, 150) ;
dans lequel ladite interprétation par la machine d'analyseur de sang (110) comprend l'analyse, en utilisant ledit algorithme, des données de résultats de test du sang à partir de la machine d'analyseur de sang et les données de résultats de test externe à partir des un ou plusieurs dispositifs d'analyseur de sang externe (130, 140, 150),
dans lequel les un ou plusieurs messages affichés par la machine d'analyseur de sang (110) sont basés sur ladite interprétation des données de résultats de test du sang à partir de la machine d'analyseur de sang (110) et les données de résultats de test externe à partir des un ou plusieurs dispositifs d'analyseur de sang externes (130, 140, 150),
l'interprétation comprenant en outre la comparaison et la mis en contraste des résultats de test l'un avec l'autre en utilisant un algorithme informatisé.

2. Procédé selon la revendication 1,
dans lequel les un ou plusieurs messages affichés par la machine d'analyseur de sang (110) sont une sorite d'interprétation de résultats qui est différente de la sortie des données de résultats de test du sang de la machine d'analyseur de sang.

3. Procédé selon la revendication 1,
dans lequel la réalisation d'un ou de plusieurs tests caractéristiques du sang par la machine d'analyseur de sang (110) sur l'échantillon de sang comprend la mesure des caractéristiques de coagulation du sang.

4. Procédé selon la revendication 1, comprenant en outre :
après l'affichage de l'un ou de plusieurs messages, la réception, à la machine d'analyseur de sang (110), d'une sélection d'utilisateur d'un élément sélectionnable pour changer un mode d'affichage de l'interface d'utilisateur (120) ; et
en réponse à la réception de la sélection de l'utilisateur, le changement d'affichage (120) de l'un ou de plusieurs messages pour afficher des informations graphiques ou numériques correspondant au moins à certaines des données de résultats de test du sang.

5. Procédé selon la revendication 1,
dans lequel les une ou plusieurs valeurs de seuil préréglées sont paramétrables par un opérateur de la machine d'analyseur de sang (110) de manière à modifier une ou plusieurs valeurs seuils par défaut à une ou plusieurs valeurs seuils paramétrées.

6. Procédé selon la revendication 1 ou 5,
dans lequel la machine d'analyseur de sang (110) comprend une console de commande comprenant un dispositif d'affichage d'interface d'utilisateur (120), un ou plusieurs processeurs d'ordinateur, et un dispositif de mémoire lisible sur ordinateur, dans lequel la pluralité d'algorithmes accessibles à la machine d'analyseur de sang (110) et les unes ou plusieurs valeurs de seuil préréglées sont stockés par le dispositif de mémoire lisible sur ordinateur.

7. Système d'analyseur de sang (100) pour fournir une sortie d'interprétation de résultats, comprenant :
une machine d'analyseur de sang (110) configurée pour produire des données de résultats de test du sang en effectuant un ou plusieurs tests caractéristiques du sang ;
une interface d'utilisateur (120) comprenant un dispositif d'affichage configuré pour afficher les données de résultats de test du sang ;
un ou plusieurs processeurs ;
un dispositif de mémoire d'ordinateur stockant des instructions lisibles sur ordinateur pour un ou plusieurs algorithmes pour interpréter les données de résultats de test du sang, dans lequel les instructions lisibles sur ordinateur, quand elles sont exécutées par les un ou plusieurs processeurs, sont configurées pour entraîner le système (100) à effectuer au moins les opérations suivantes :
analyser les données de résultats de test du sang ; et
sortir via l'interface d'utilisateur (120) u ou plusieurs messages indicateurs d'un diagnostic potentiel ou d'un état sanitaire basé sur l'analyse des données de résultats de test du sang ;
dans lequel
les instructions lisibles sur ordinateur, quand elle sont exécutées par les un ou plusieurs processeurs, sont configurées pour entraîner le système (100) à inciter un utilisateur pour une entrée indicatrice d'un paramètre clinique d'un patient ; et
les instructions lisibles sur ordinateur, quand elles sont exécutées par les un ou plusieurs processeurs, sont configurées pour entraîner le système (100) à sélectionner un algorithme particulier d'une pluralité de algorithmes pour interpréter les données de résultats de test du sang en réponse à l'entrée de l'utilisateur indicatrice du paramètre clinique du patient ;
**caractérisé en ce que**
ladite analyse des données de résultats de test du sang comprend l'analyse des données de résultats de test du sang en comparaison à une ou plusieurs valeurs de seuil spécifiques d'algorithme préréglées dans une matrice de risque (270) en utilisant l'algorithme pour sortir les dits un ou plusieurs messages ;
dans lequel les instructions lisibles sur ordinateur, quand elle sont exécutées par les un ou plusieurs processeurs, sont configurées pour entraîner le système (100) à recevoir les données de résultats de test externe à partir d'un ou de plusieurs dispositifs d'analyseur de sang externes (130, 140, 150) qui sont différents de et distants dudit système d'analyseur de sang (100), dans lequel le système d'analyseur de sang (100) comprend une connexion de données pour recevoir les données de résultats de test externe des un ou plusieurs dispositifs d'analyseur de sang externes (130, 140, 150), la connexion de données comprenant une connexion de câble de données ou une connexion de réseau sans fil (102) ; et
dans lequel ladite interprétation par la machine d'analyseur de sang (110) comprend l'analyse, en utilisant ledit algorithme, des données de résultats de test du sang de la machine d'analyseur de sang et des données de résultats de test externe de l'un ou plusieurs dispositifs d'analyseur de sang externes (130, 140, 150), dans lequel l'un ou plusieurs messages affichés par la machine d'analyseur de sang (110) sont basés sur ladite interprétation des données de résultats de test du sang de la machine d'analyseur de sang (110) et les données de résultats de test externe de l'un ou plusieurs dispositifs d'analyseur de sang externes (130, 140, 150),
l'interprétation comprenant en outre la comparaison et la mise en contraste des résultats de test l'un avec l'autre en utilisant un algorithme informatisé.

8. Système (100) selon la revendication 7,
dans lequel le dispositif de test de sang (110) est un dispositif de test de thromboélastométrie (110) comprenant une sonde amovible pour interagir avec au moins une partie d'un échantillon de sang pour mesurer les caractéristiques de coagulation du sang de l'échantillon de sang ; et dans lequel les données de résultats de test du sang sont indicatrices des caractéristiques de coagulation du sang.

9. Système (100) selon la revendication 7 ou 8,
dans lequel les un ou plusieurs messages indicateurs d'un diagnostic potentiel ou d'un état sanitaire sont une sortie d'interprétation de résultats qui est affichée par le dispositif d'affichage et qui est différente des données de résultats de test du sang.
